Europäisches Patentamt

(19) European Patent Office

Office européen des brevets

(11) Publication number: **0 281 493 B1**

## (12) EUROPEAN PATENT SPECIFICATION

(45) Date of publication of patent specification:
11.09.91 Bulletin 91/37

(51) Int. Cl.⁵: **G01N 33/543, G01N 33/569, G01N 33/546**

(21) Application number: 88500020.8

(22) Date of filing: 25.02.88

(54) A process for the preparation of diagnostic reagents.

(30) Priority: 02.03.87 ES 8700552

(43) Date of publication of application:
07.09.88 Bulletin 88/36

(45) Publication of the grant of the patent:
11.09.91 Bulletin 91/37

(84) Designated Contracting States:
BE CH DE ES FR GB IT LI

(56) References cited:
EP-A- 0 089 598
EP-A- 0 123 901
EP-A- 0 131 546
US-A- 4 329 151
CHEMICAL ABSTRACTS, vol. 93, no. 3, 21 July
1980, Columbus, OH (US); p. 540, no. 24332f

(56) References cited:
CHEMICAL ABSTRACTS, vol. 72, no. 17, 27
April 1970, Columbus, OH (US); G.L.BACH et
al., p. 194, no. 88295v
CHEMICAL ABSTRACTS, vol. 94, no. 9, 02
March 1981, Columbus, OH (US);
B.A.MARCHLEWICZ et al., p. 340, no. 61248z

(73) Proprietor: LABORATORIOS
KNICKERBOCKER S.A.E.
Comte Borrell 158
E-08015 Barcelona (ES)

(72) Inventor: Capdevila Moragas, Maria José
Comte Borrell 158
E-08015 Barcelona (ES)

(74) Representative: Curell Sunol, Jorge et al
c/o Dr. Ing. M. Curell Sunol I.I. S.L. Passeig de
Gràcia 65 bis
E-08008 Barcelona (ES)

## Description

This invention relates to a process for the preparation of diagnostic reagents for an agglutination immuno assay comprising the steps of : preparing a solution of proteins chosen from the group formed by antigens and antibodies ; providing a suspension of a particulate material, subjecting said proteins to stabilization with a bifunctional chemical agent ; and sensitizing said particulate material by adsorption of said proteins thereon.

When a bacterium or other microorganism infects and animal, there is an immunological response evidenced by the appearance of specific antibodies in the infected animal's blood. The antibodies are proteins capable of recognising and binding specifically to the molecules foreign to the organism stimulating the production thereof (antigens). Generally such antibodies recognise a part of the invading microorganism. Frequently also antibodies are produced which recognise proteins produced and excreted by the infecting microorganism.

Thus, for example, when a human being or other animal is infected by streptococci, on developing in the host organism, the microorganism produces over 20 different exocellular proteins, many of them toxic. The best known among them are streptolysin O, streptodornase, streptokinase, hyaluronidase, streptoprotease and nicotinamido adenine dinucleotide glycohydrolase. All of them stimulate the production of specific antibodies in the host organism.

A correct and speedy detection of the streptococcal infections is of clinical importance for their relationship with rheumatic fever, acute glomerulonephritis, rheumatoid arthritis and erythema nodosum.

A frequently used form for diagnosing or confirming the diagnosis of this and other infections is the detection in the blood serum of high levels of antibodies specific to the invading microorganism or to one or more of the exocellular proteins produced. In the case of streptococcus, the most frequent determination is the level of anti-streptolysin O antibodies. Similarly, but inversely, it is also possible to identify the antigen in the blood serum in certain cases.

A fairly common way of showing high levels of anti-streptolysin O and other specific antibodies is use of the so-called agglutination tests.

In this type of test, the antigen (for example, the streptolysin O) is adsorbed on a carrier particle. The thus "sensitized" particles are in a homogenous suspension in an appropriate liquid medium. When the suspension of sensitized particles is contacted with a serum containing a sufficient amount of the specific antibody, the antibody binds to the adsorbed antigen, causing an agglutination of the particles which is obvious to the naked eye in view of the macroscopic change in the appearance of the suspension. These tests are, therefore, an extraordinarily simple and speedy way for detecting the presence of certain antibodies.

Various particulate materials such as red blood cells, synthetic resins, collodion, bentonite, quartz and polystyrene latex have been used for agglutination tests. Among these, the polystyrene latex particles seem to be the most appropriate for their spherical form, their uniform size and their capacity of adsorbing many proteins by hydrophobic forces.

Nevertheless, the agglutination tests have several serious drawbacks. In the first place, the adsorption of the proteins on the particulate material is not permanent, whereby the amount of bound protein diminishes progressively with the time elapsed after preparation of the sensitized latex. Thus, the latex loses "sensitivity", with a progressively larger amount of antibody being required to produce agglutination.

A further important drawback of the agglutination tests is the fact that all animal sera frequently contain small amounts of specific antibody, even in the absence of an actual infection. These levels are known and are called "normal", such that only levels above the normal levels are indicative of a recent infection. The presence of these normal specific antibody levels makes a prior dilution of all the sera necessary, to prevent the agglutination of the sensitized latex with all of them. In this way, only the sera containing specific antibody counts above the normal levels will cause agglutination of the sensitized latex. The prior dilution of a serum is a drawback, because it increases the laboratory work and may be a source of errors and inaccuracies.

A third drawback of the agglutination tests is the existence of the so-called "zone phenomenon". When the amount of specific antibody in the analysed sample is very high, it frequently happens that each adsorbed antigen molecule binds to one antibody molecule. In this way, the formation of bonds between one same antibody molecule with two antigen molecules bonded to different particles is prevented and three is no agglutination in spite of high levels of specific antibody. The zone phenomenon is more frequent when the particulate material is sensitized with small amounts of antigen, a regular practice followed to avoid excessive sample dilutions or inspecific agglutinations.

In this sense, EP-A-0089598 may be cited. This relates to an anti-streptolysin-O-latex reagent, in the preparation of which the streptolysin is pretreated with a low molecular bifunctional chemical agent and, if necessary, with gamma-globulin or Fab fragments of gammaglobulin, followed by the coupling of streptolysin to the latex particles. Nevertheless it makes no reference to any streptolysin remaining in solution nor to the intentional addition of an excess of streptolysin that remains unabsorbed to the latex particles and stable in the solution.

When a specific antibody is adsorbed on the particulate material (latex), instead of an antigen, the agglutination test system described may be applied for the detection of antigens (instead of antibodies). The antigen determination test has the same drawbacks as stated above for the detection of antibodies.

The object of the invention is to prepare a diagnostic reagent which ray be used in the detection of antibodies or antigens without needing prior dilution of the sample to be analysed and showing a substantially reduced zone phenomenon.

This is attained with a process of the type stated hereinbefore and wherein said sensitization step is carried out with an excess of said stabilized proteins such that a fraction of the proteins remains unadsorbed and stable in the solution, this unbound fraction of antigen or antibody being properly adjusted so as to neutralise the desired levels of specific antibodies or antigens present in the samples to be analysed, thereby avoiding the need to dilute such samples previously.

In one development of the invention, said step of preparing the protein solution comprises the step of culturing a streptococcus in a culture medium, producing exoproteins ; a step of separating the streptococcus from the culture medium ; a step of purification and concentration of said proteins, said preparation step being followed by the steps of providing particulate material, stabilization and sensitization, in which the excess of stabilized exoproteins is such that a fraction of unadsorbed exoproteins capable of neutralising between 150 and 200 UI/ml of antistreptolysin O in a mixture of equal portions of reagent and sample to be analysed remains in solution.

Preferably according to the invention said separation step is carried out by centrifugation.

Optionally according to the invention, said purification and concentration step may be carried out by precipitation with 75% saturated ammonium sulfate.

Preferably according to the invention, said bifunctional chemical agent used in said stabilisation step is glutardialdehyde, which is added to a concentrated streptococcus exoprotein solution containing from 60 to 120 g/l of protein and from 5 to 10 g/l of glycine, the added glutardialdehyde being 0.5% by weight of the solution.

The particulate material providing the best results is formed by polystyrene latex particles.

The invention also relates to the use of the reagents prepared by the process described, for diagnosing infections in human beings or animals.

The antigens or antibodies are stabilized by intermolecular cross-linking in an appropriate medium and in presence of precise concentrations of a protein cross-linking chemical agent such as glutardialdehyde. The thus treated antigen or antibody is adsorbed on the particulate material (latex) and retains its capacity of binding to specific antibodies or antigens. The incorporation of excess treated antigen or antibody in the latex suspension allows a fraction of antigen or antibody not bound to the particulate to be obtained, which fraction is maintained with extraordinary stability. This unbound fraction of antigen or antibody is properly adjusted so as to neutralise the desired levels of specific antibodies or antigens present in the samples to be analysed, thereby avoiding the need to dilute such samples previously. Another significant advantage of the invention is that, with it being possible to sensitize the latex strongly, the zone phenomenon is substantially reduced or cancelled out.

Therefore, the invention represents a significant advance in the present state of the art of specific antigen or antibody agglutination tests, allowing the preparation of diagnostic reagents : a) having extraordinary stability ; b) not requiring prior dilution of the sample and c) reducing or cancelling out the zone phenomenon.

To prepare a reagent for the detection of streptococcus infections, the sequence outlined below may be followed :

In the first place, there is prepared a purified mixture of streptococcus exoproteins as follows : betahemolytic streptococcus is cultured in an appropriate culture medium containing from 1 to 10 g/l of glucose, other nutrients required and a pH of 6 to 8, for the appropriate period of time to obtain a maximum production of exoproteins (usually from 8 to 24 hours). At the end of this time, the microorganism is removed by centrifugation and the streptococcus exoproteins in the culture broth are purified. The purification is effected by precipitation with 75% saturated ammonium sulphate, the centrifuged protein material being resuspended with a small amount of buffer. The mixture of exoproteins is purified and concentrated in this way.

The streptococcus exoprotein concentrate is stabilised thereafter by the addition of a bifunctional chemical cross-linking agent, preferably with 0.1 to 1.0% of glutardialdehyde.

Finally, the diagnostic formulation is prepared by mixing the stabilised exoprotein concentrate with a latex suspension. First the latex is sensitized by adsorption of the protein material on the particle surface at room temperature. When the particles have adsorbed the maximum amount of proteins possible, the addition of stabilized exoproteins is continued, these remaining in solution, up to the amount required to neutralise the normal levels of specific antibodies present in a sample to be analysed (normally human serum).

The reagent prepared according to this process is superior to those of the current state of the art, since it no longer requires the prior dilution of the serum to be tested so as correctly to discern between normal sera and sera of infected patients and it is stable (maintaining all its characteristics) for a minimum of 18 months at refrigerator temperature.

The following non-limiting inventions illustrate the invention :

## EXAMPLE 1

In the first place there was prepared a solution of streptococcus exoproteins containing, per litre : 10 g NaCl, 7.5 g glycine, 3 g bovine serum albumin, 0.5 g sodium dithionite, 1 g sodium azide, 3.4 g disodium ethylenediamine tetraacetate dihydrate and 80 g of protein obtained by culturing streptococcus and concentrated by precipitation with ammonium sulfate in such a way as to contain a streptolysin O concentration ranging from 450 to 500 UI/ml. The pH of the solution was adjusted to 8.2.

To the above solution there was added 0.5% glutardialdehyde at room temperature and the mixture was stirred for 30 minutes.

Separately there was prepared a latex suspension containing 95 mM glycine, 0.17 M NaCl, 0.3% bovine serum albumin, 2.9 mM sodium dithionite, 15.4 mM sodium azide, 28% 0.81 μ latex.

Thereafter 750 ml of the latex suspension was mixed with 950 ml of the glutardialdehyde treated streptococcus exoprotein solution. The mixture was made by slowly adding the second solution over the first one, with continuous stirring at room temperature. Finally, 300 ml of a solution of 95 mM glycine, 0.17 M NaCl, 0.3% bovine serum albumin, 2.9 mM sodium dithionite, 15.4 mM sodium azide were added.

0.19% glutardialdehyde and 1.5 g sodium borohydride were added to the sensitized latex suspension thus obtained.

In this way there was prepared a diagnostic reagent characterised by its capacity to agglutinate when one drop of reagent is mixed with a drop of serum containing an anti-streptolysin level in excess of 150 UI/ml, while it does not agglutinate when the serum contains a streptolysin concentration equal to or less than 150 UI/ml.

## EXAMPLE 2

A solution of streptococcus exoproteins obtained by precipitation of the streptococcus free culture medium with 75% saturated ammonium sulfate and resuspension of the precipitate with a small volume of 95 mM glycine such that the streptolysin O content ranged from 450 to 500 UI/ml was prepared. To this solution there was added 0.5 part % of glutardialdehyde at room temperature and the mixture was shaken for a few minutes.

There was also prepared a latex suspension containing 95 mM glycine, 0.17 M NaCl, 15.4 mM sodium azide and 28% 0.81 μ latex.

A diagnostic reagent was prepared from the glutardialdehyde treated streptococcus exoprotein solution and the latex suspension, in the same way as described in Example 1.

## EXAMPLE 3

A glutardialdehyde treated streptococcus exoprotein solution was prepared as described in Example 1 or 2, together with a latex suspension containing 95 mM glycine, 0.17 M NaCl, 0.3% bovine serum albumin, 2.9 mM sodium dithionite, 15.4 mM sodium azide and 28% 0.81 μ latex.

A diagnostic reagent was prepared from the glutardialdehyde treated streptococcus exoprotein solution and the latex suspension in the same way as described in Example 1.

## Claims

1. A process for the preparation of diagnostic reagents for an agglutination immuno assay comprising the steps of : preparing a solution of proteins chosen from the group formed by antigens and antibodies ; providing a suspension of a particulate material ; subjecting said proteins to stabilization with a bifunctional chemical agent and sensitizing said particulate material by adsorption of the said proteins thereon, characterized by said sensitization step is carried out with an excess of said stabilized proteins such that a fraction of the proteins remains unadsorbed and stable in the solution, this unbound fraction of antigen or antibody being properly adjusted so as to neutralise the desired levels of specific antibodies or antigens present in the samples to be analysed, thereby avoiding the need to dilute such samples previously.

2. The process of claim 1, wherein said step of preparing the protein solution comprises the step of culturing a streptococcus in a culture medium, producing exoproteins ; a step of separating the streptococcus from the culture medium ; a step of purification and concentration of said proteins, said preparation step being followed by the steps of providing particulate material, stabilization and sensitization, in which the excess of stabilized exoproteins is such that a fraction of unadsorbed exoproteins capable of neutralising between 150 and 200 UI/ml of antistreptolysin O in a mixture of equal portions of reagent and sample to be analysed remains in solution.

3. The process of claim 2, wherein said streptococcus is beta-hemolytic.

4. The process of claim 2, wherein said separation step is carried out by centrifugation.

5. The process of claim 2, wherein said purification and concentration step is carried out by precipitation with 75% saturated ammonium sulfate.

6. The process of claim 2, wherein said bifunctional chemical agent used in said stabilization step is glutardialdehyde.

7. The process of claim 6, wherein said glutardialdehyde is added to a concentrated streptococcus exoprotein solution containing from 60 to 120 g/l of protein and from 5 to 10 g/l of glycine, with the added glutardialdehyde being 0.5% by weight of the solution.

8. The process of claim 2, wherein said particulate material is formed by polystyrene latex particles.

9. The use of the reagents prepared by the process according to any one of the previous claims for diagnosing infections in human beings or animals.

10. Diagnostic reagent obtainable by a process according to claims 1 to 8.

## Patentansprüche

1. Verfahren zur Herstellung von diagnostischen Reagenzien für ein Agglutinations-immuno-assay mit folgenden Schritten : Herstellen einer Proteinlösung aus der Gruppe Antigene und Antikörper ; Zurverfügungstellung einer Suspension eines teilchenförmigen Materials ; Stabilisierung der Proteine mit einem bifunktionalen chemischen Agens und Sensibilisierung des teilchenförmigen Materials durch Adsorption der genannten Proteine auf dem Material, dadurch gekennzeichnet, daß der Sensibilisierungsschritt mit einem Überschuß an den genannten stabilisierten Proteinen so durchgeführt wird, daß eine Fraktion der Proteine nicht adsorbiert wird und stabil in der Lösung verbleibt, wobei diese ungebundene Fraktion des Antigens oder Antikörpers genau so dosiert wird, um die gewünschte Menge spezifischer Antikörper oder Antigene, die in den zu analysierenden Proben vorhanden sind, unter gleichzeitiger Vermeidung der Notwendigkeit, solche Proben zuvor zu verdünnen, zu neutralisieren.

2. Verfahren nach Anspruch 1, bei dem der Herstellungsschritt für die Proteinlösung die Kultivierung eines Streptokokkus in einem Kulturmedium, das Exoproteine produziert, einen Schritt zur Trennung des Streptokokkus vom Kulturmedium und eine Reinigungs- und Konzentrationsstufe des Proteins umfaßt, an die sich Verfahrensschritte zur Herstellung des teilchenförmigen Materials, zur Stabilisierung und Sensibilisierung anschliessen, wobei der Überschuß der stabilisierten Exoproteine derart ist, daß eine Fraktion der nicht adsorbierten Exoproteine in Lösung verbleibt, die in der Lage ist, 150 bis 200 UI/ml von Antistreptolysin O in einer Mischung von gleichen Teilen Reagens und zu analysierender Probe zu neutralisieren.

3. Verfahren nach Anspruch 2, bei dem der Streptokokkus β-hämolytisch ist.

4. Verfahren nach Anspruch 2, bei dem der Trennschritt durch Zentrifugieren ausgeführt wird.

5. Verfahren nach Anspruch 2, bei dem die Reinigungs- und Konzentrationsstufe durch Präzipitation mit 75 prozentigem Ammoniumsulfat durchgeführt wird.

6. Verfahren nach Anspruch 2, bei dem das bifunktionelle chemische Agens, das im Stabilisierungsschritt genutzt wird, Glutardialdehyd ist.

7. Verfahren nach Anspruch 6, bei dem das Glutardialdehyd zu einer konzentrierten Streptokokkusexoproteinlösung, die 60 bis 120 g/l Protein und 5 bis 10 g/l Glycin enthält, zugegeben wird, wobei das zugegebene Glutardialdehyd 0,5 Gew.-% der Lösung ausmacht.

8. Verfahren nach Anspruch 2, bei dem das teilchenförmige Material von Polystyrenlatexteilchen gebildet wird.

9. Verwendung der Reagenzien, hergestellt nach dem Verfahren gemäß einem der vorstehenden Ansprüche zur Diagnose von Infektionen bei Menschen oder Tieren.

10. Diagnostisches Reagens, hergestellt nach einem Verfahren gemäß den Ansprüchen 1 bis 8.

## Revendications

1. Procédé pour la préparation de réactifs diagnostiques pour un test immunologique d'agglutination comprenant les étapes consistant à : préparer une solution de protéines choisies parmi le groupe formé par des antigènes et des anticorps ; fournir une suspension d'une matière particulaire ; soumettre les protéines à la stabilisation avec un agent chimique bifonctionnel et sensibiliser la matière particulaire par adsorption des protéines sur celle-ci, caractérisé par le fait que l'étape de sensibilisation est effectuée avec un excédent de protéines stabilisées de façon qu'une fraction des protéines demeure non adsorbée et stable dans la solution, cette fraction non liée d'antigènes ou d'anticorps étant correctement ajustée de façon à neutraliser les taux souhaités d'anticorps ou d'antigènes spécifiques présents dans les échantillons à analyser, ce qui permet d'éviter d'avoir à diluer préalablement ces échantillons.

2. Procédé selon la revendication 1, dans lequel la préparation de la solution comprend l'étape consistant à mettre en culture un streptocoque dans un milieu de culture, produisant des exoprotéines ; une étape de séparation du streptocoque du milieu de culture ; une étape de purification et de concentration des protéines, l'étape de préparation étant suivie des étapes consistant à fournir la matière particulaire, à effectuer la stabilisation et la sensibilisation, opérations au cours desquelles l'excédent d'exoprotéines stabilisées est tel qu'une fraction d'exoprotéines non adsorbées capable de neutraliser entre 150 et 200 UI/ml d'antistreptolysine O dans un mélange de portions égales de réactif et d'échantillon à analyser reste en solution.

3. Procédé selon la revendication 2, dans lequel le streptocoque est béta-hémolytique.

4. Procédé selon la revendication 2, dans lequel l'étape de séparation s'effectue par centrifugation.

5. Procédé selon la revendication 2, dans lequel l'étape de purification et de concentration s'effectue par précipitation avec du sulfate d'ammonium saturé à 75%.

6. Procédé selon la revendication 2, dans lequel l'agent chimique bifonctionnel utilisé dans l'étape de stabilisation est le glutardialdéhyde.

7. Procédé selon la revendication 6, dans lequel le glutardialdéhyde est ajouté à une solution d'exoprotéines de streptocoque concentrée contenant de 60 à 120 g/l de protéines et de 5 à 10 g/l de glycine, avec le glutardialdéhyde ajouté constituant 0,5% en poids de la solution.

8. Procédé selon la revendication 2, dans lequel la matière particulaire est formée par des particules de latex de polyatyrène.

9. Utilisation des réactifs préparés par le procédé selon l'une quelconque des revendications précédentes pour diagnostiquer des infections chez l'homme ou l'animal.

10. Réactif de diagnostic pouvant être obtenu par un procédé selon les revendications 1 à 8.